(19) Europäisches Patentamt
European Patent Office
Office européen des brevets

(11) Veröffentlichungsnummer: **0 039 860**
**A1**

(12) # EUROPÄISCHE PATENTANMELDUNG

(21) Anmeldenummer: **81103330.7**

(22) Anmeldetag: **02.05.81**

(51) Int. Cl.³: **A 61 K 7/08**
A 61 K 7/50, C 11 D 1/42

(30) Priorität: **09.05.80 DE 3017814**

(43) Veröffentlichungstag der Anmeldung:
**18.11.81 Patentblatt 81/46**

(84) Benannte Vertragsstaaten:
**AT BE CH DE FR GB IT LI LU NL SE**

(71) Anmelder: **Henkel Kommanditgesellschaft auf Aktien**
**Postfach 1100 Henkelstrasse 67**
**D-4000 Düsseldorf 1(DE)**

(72) Erfinder: **Maak, Norbert, Dr.**
**Liebigstrasse 18**
**D-4040 Neuss(DE)**

(72) Erfinder: **Stader, Wolfgang**
**Gärtnerstrasse 37**
**D-5650 Solingen(DE)**

(72) Erfinder: **Fischer, Herbert, Dr.**
**Kamper Weg 139**
**D-4000 Düsseldorf 12(DE)**

(54) **Neuartige Tenside, deren Herstellung und Verwendung in kosmetischen Reinigungsmitteln sowie diese enthaltende hautfreundliche, kosmetische Reinigungsmittel.**

(57) Die neuartigen Tenside auf Basis von Salzen anionischer Tenside, enthalten als Gegenion ein reduktiv aminiertes Derivat reduzierender Mono-, Di- oder Oligosaccharide der allgemeinen Formel (I)

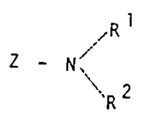

(I)

$R^1$, $R^2$ = gleich oder verschieden H, Alkyl- oder Hydroxyalkyl-($C_{1-4}$)Z = Polyhydroxyalkyl-(C ≤ ), ggf. mit einem Mono-, Di- oder Oligosaccharid glukosidisch verknüpft. In den Formulierungen bevorzugte anionische Tenside sind Alkylschwefelsäuremonoester bzw. Alkyletherschwefelsäuremonoester (Alkyl-$C_{8-18}$) besonders Fettalkohol-$C_{12-14}$)-etherschwefelsäuremonoester mit ca. 2 Ethylenoxidgruppen im Molekül.

Das als Gegenion dienende Zuckerderivat leitet sich vorzugsweise von Glucose ab und ist insbesonder N-Methylglucamin, N-β-Hydroxyethylglucamin bzw. N-β-Hydroxyethyl-N-methylglucamin.

Bei der Herstellung der neuartigen Tenside werden die nach bekannten Verfahren erhaltenen Zuckerderivate mit der äquivalenten Menge des sauren anionischen Tensids neutralisiert.

Die Produkte zeichnen sich durch besonders hautschonende Eigenschaften aus. In hautfreundlichen kosmetischen Reinigungsmitteln können die neuen Tenside alleine oder in Kombination mit anderen Tensiden in Mengen von 10-70 Gew.% eingesetzt werden.

**EP 0 039 860 A1**

Croydon Printing Company Ltd.

COMPLETE DOCUMENT

0039860

HENKEL KGaA
ZR-FE/Patente
z-sü

D-4000 Düsseldorf, den 7.5.1980

# Patentanmeldung

D 6123 EP

"Neuartige Tenside, deren Herstellung und Verwendung in kosmetischen Reinigungsmitteln sowie diese enthaltende hautfreundliche,kosmetische Reinigungsmittel"

Gegenstand der Erfindung sind neuartige Tenside auf Basis von Salzen anionischer Tenside, insbesondere Alkylsulfate und Alkylethersulfate, die als Gegenion ein reduktiv aminiertes Derivat reduzierender Mono-, Di- oder Oligosaccharide enthalten, deren Herstellung und Verwendung in kosmetischen Reinigungsmitteln, sowie diese enthaltende hautfreundliche,kosmetische Reinigungsmittel.

Die Bemühungen,keratinhaltiges Material wie Haut und Haare vor den schädlichen Wirkungen von Detergentien zu schützen, wurden bereits vor langer Zeit aufgenommen, ohne daß es bis heute zu einer völlig befriedigenden Lösung gekommen wäre. Es wurden inzwischen zahlreiche Möglichkeiten zur Lösung dieser Probleme vorgeschlagen, wobei für die Aniontenside, die am verbreitesten eingesetzte Tensidklasse, insbesondere zwei unterschiedliche Lösungswege bearbeitet wurden. Der erste Lösungsweg besteht in dem Zusatz von die Hautfreundlichkeit verbessernden Produkten zu den Aniontensiden,wie insbesondere Alkylsulfate oder Alkylethersulfate in Form ihrer Alkali-, Amin- oder Alkylolaminsalze enthaltenden Reinigungsmitteln. Als solche, die Hautfreundlichkeit verbessernden Produkte, finden in erster Linie mehr oder weniger chemisch modifizierte Eiweißkörper, jedweder Provenienz Verwendung.

Der zweite Lösungsweg zur Verbesserung der Hautfreundlichkeit von Aniontensiden, insbesondere von Alkylsulfaten und Alkylethersulfaten ist die Verwendung anderer Gegenionen anstelle von Natrium. Hierbei haben sich insbesondere die Di- und Triethanolaminsalze der Alkyl- und Alkylethersulfate durchsetzen können, die hautschonendere Eigenschaften aufweisen, als die entsprechenden Natriumsalze.

Es wurde nun gefunden, daß neuartige Tenside auf Basis von Salzen anionischer Tenside, die als Gegenion ein durch reduktive Aminierung reduzierender Mono-, Di- oder Oligosaccharide gewonnenes Zuckerderivat der allgemeinen Formel I enthalten

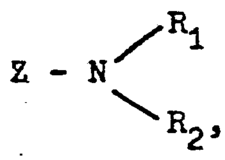 I

in der $R_1$ und $R_2$, die gleich oder verschieden sein können und Wasserstoff, einen Alkylrest oder Hydroxyalkylrest mit 1 - 4 Kohlenstoffatomen darstellen, während Z einen Polyhydroxyalkylrest mit bis zu 7 Kohlenstoffatomen bedeutet, der gegebenenfalls mit einem Mono-, Di- oder Oligosaccharid glukosidisch verknüpft ist, sich durch besonders hautschonende Eigenschaften auszeichnen.

Besondere Bedeutung kommt dabei denjenigen neuartigen Tensiden zu, deren als Gegenion dienendes Zuckerderivat sich von der Glucose ableitet und insbesondere N-Methylglucamin, N-β-Hydroxyethylglucamin, beziehungsweise N-β-Hydroxyethyl-N-methylglucamin ist.

Als saure anionische Tenside, die durch Neutralisation mit den vorgenannten Zuckerderivaten zu den neuartigen Tensiden führen, sind in erster Linie organische Schwefelsäurereaktionsprodukte zu nennen, die in ihrem Molekül neben einer Sulfonsäure- oder Schwefelsäureestergruppe eine Alkylgruppe mit 8 bis 22 Kohlenstoffatomen, eine Alkylbenzolgruppe mit 9 bis 15 Kohlenstöffatomen in der Alkylgruppe, eine Alkylglycerylethergruppe mit höherem Alkylrest, eine Fettsäuremonoglyceridgruppe, eine Alkylphenolethylenoxidethergruppe mit 1 bis 12 Ethylenoxideinheiten und einem Alkylrest mit 8 bis 18 Kohlenstoffatomen, eine Alkylethylenoxidethergruppe mit 1 bis 30 Ethylenoxideinheiten und einem Alkylrest mit 8 bis 24 Kohlenstoffatomen enthalten. Besondere Bedeutung kommt dabei den Alkylschwefelsäuremonoestern, beziehungsweise Alkyletherschwefelsäuremonoestern mit einer Alkylkette von 8 bis 18 Kohlensotffatomen zu und unter diesen wiederum dem Fettalkohol-$C_{12}$-$C_{14}$-etherschwefelsäuremonoester mit circa 2 Ethylenoxidgruppen im Molekül.

Als Ausgangsmaterialien, die nach dem allgemein bekannten Verfahren der reduktiven Aminierung zu den als Gegenion der Aniontenside erfindungsgemäß einzusetzenden Zuckerderivaten führen, sind zum Beispiel Glucose, Galactose, Maltose, Lactose, Cellobiose, Maltotriose, Maltodextrine sowie sonstige reduzierende Stärkeabbauprodukte wie Glucosesirup zu nennen.

Als Aminierungskomponente für die Herstellung der erfindungsgemäß als Gegenion dienenden Zuckerderivate kommen neben Ammoniak zum Beispiel Methylamin, Ethylamin, Propylamin, Butylamin, Dimethylamin, Diethylamin, Ethanolamin, Diethanolamin in Frage.

Die Herstellung der erfindungsgemäßen neuartigen Tenside erfolgt dadurch, daß man die nach bekannten Verfahren durch reduktive Aminierung von Mono-, Di- oder Oligosacchariden, wie sie zum Beispiel in den amerikanischen Patentschriften 2 016 962 und 2 830 983 beschrieben ist, gewonnenen Zuckerderivate mit der äquivalenten Menge an Alkylschwefelsäuremonoester, beziehungsweise Alkyletherschwefelsäureester oder einem der anderen genannten Aniontenside neutralisiert. Bevorzugte erfindungsgemäße Tenside sind demnach Neutralsalze von Alkylschwefelsäuremonoestern, beziehungweise Alkyletherschwefelsäuremonoestern, deren Alkylkette 8 bis 18 Kohlenstoffatome besitzt mit N-Methylglucamin, N-ß-Hydroxyethyl-N-methylglucamin, wobei den Neutralsalzen des Fettalkohol-$C_{12}$-$C_{14}$-ethersulfats mit circa 2 Ethylenoxidgruppen im Molekül besondere Bedeutung zukommt.

Ein weiterer Gegenstand der Erfindung ist die Verwendung der erfindungsgemäßen neuartigen Tenside in hautfreundlichen kosmetischen Reinigungsmitteln wie Badezusätzen, Duschbadzusätzen, Haarshampoos, Babyshampoos. Die erfindungsgemäßen neuartigen Tenside werden in den genannten Produkten anstelle der bisher verwendeten Tenside in üblicher Weise eingesetzt.

Kosmetische Reinigungsmittel, die die erfindungsgemäßen Tenside enthalten, stellen einen weiteren Gegenstand der Erfindung dar. Der Gehalt dieser hautfreundlichen Handwaschmittel, Badezusätze, Duschbadzusätze, Haarshampoos, Babyshampoos an den erfindungsgemäßen Tensiden bewegt sich im allgemeinen zwischen 10 bis 70 Gewichtsprozent, bezogen auf die gesamte Zubereitung. Daneben enthalten die Mittel, die in derartigen Produkten üblichen Bestandteile. Außer durch ihre herausragende Hautfreundlichkeit erweisen sich die erfindungsgemäßen

Tenside als Rohstoffe für die kosmetischen Zubereitungen besonders geeignet, da sie in Wasser relativ niedrigviskose Lösungen ergeben, die sich mit Natriumchlorid oder Fettsäurealkanolamiden gut verdicken lassen, sehr gut schäumen, mit den übrigen Bestandteilen verträglich sind, sich sehr gut in die kosmetischen Zubereitungen einarbeiten lassen und lagerstabil sind.

Die nachfolgenden Beispiele sollen den Erfindungsgegenstand näher erläutern, ohne ihn jedoch hierauf zu beschränken.

/6

## Beispiele

### Produkt A

**Herstellung des Neutralisationsproduktes aus N-β-Hydroxyethyl-N-methylglucamin und Fettalkohol-$C_{12}$-$C_{14}$-ethermonoschwefelsäureester mit circa 2 Mol Ethylenoxid**

239 g (1 Mol) N-β-Hydroxyethyl-N-methyl-glucamin wurden in 1500 ml Wasser gelöst und unter starkem Rühren mit 345 g (1 Mol) Fettalkohol-$C_{12}$-$C_{14}$-ether-monoschwefelsäureester mit 2 Mol Ethylenoxid versetzt. Das resultierende erfindungsgemäße Tensid hatte einen pH-Wert von 6,5 - 7. Die erhaltene Lösung ist etwa 28 %ig an Neutralisationsprodukt und kann in dieser Form in den verschiedensten kosmetischen Zubereitungen eingesetzt werden.

### Produkt B

**Herstellung des Neutralisationsproduktes aus N-Methylglucamin und Fettalkohol-$C_{12}$-$C_{14}$-ethermonoschwefelsäureester mit circa 2 Mol Ethylenoxid**

195 g (1 Mol) N-Methylglucamin wurden in 1500 ml Wasser gelöst und unter starkem Rühren mit 354 g (1 Mol) Fettalkohol-$C_{12}$-$C_{14}$-ether-monoschwefelsäureester mit 2 Mol Ethylenoxid versetzt. Das resultierende erfindungsgemäße Tensid hatte einen pH-Wert von 6,5 - 7. Die erhaltene Lösung ist ca. 27 %ig an Neutralisationsprodukt und kann in dieser Form in den kosmetischen Zubereitungen eingesetzt werden.

Zur Bestimmung der Hautfreundlichkeit der erfindungsgemäßen neuartigen Tenside wurde der Zein-Test durchgeführt. Als Testprodukt diente das Neutralisationsprodukt von Fettalkohol-$C_{12}$-$C_{14}$-ether-monoschwefelsäureester mit circa 2 Mol Ethylenoxid mit N-ß-Hydroxyethyl-N-methylglucamin (Produkt A). Als Vergleichssubstanzen dienten Nonylbenzolsulfonat (Na-Salz), Natriumlaurylsulfat und Natrium-Fettalkohol-$C_{12}$-$C_{14}$-ethersulfat mit 2-Ethylenoxidgruppen im Molekül.

Die bei dem Zeintest erhaltene Zeinzahl gibt an, wieviel mg Stickstoff in 100 ml einer Tensidlösung enthalten sind, wenn diese 1 Stunde lang bei 35° C mit 2 g Zein versetzt wird. Je niedriger die Zeinzahl, um so besser ist die Hautverträglichieit des Produktes. Der Zein-Test wird in der nachfolgend beschriebenen Weise durchgeführt:

1. Ansetzen der Stammlösungen:

1.1. Natronlauge 1/20 n

Die 1/20 n Natronlauge wird mit Benzoesäure (DAB 6) eingestellt. Dazu werden circa 40 bis 50 mg Benzoesäure in einen 100 ml Erlenmeyerkolben eingewogen und in ungefähr 25 ml neutralisiertem Ethylalkohol gelöst. Zum Neutralisieren wird der Alkohol mit einigen Tropfen verdünnter Natronlauge gegen Phenolphthalein bis zur bleibenden Rosafärbung versetzt. Die gelöste Benzoesäure wird nun mit der 1/20 n Natronlauge bis zur bleibenden Rosafärbung titriert und der Faktor berechnet:

1 ml 1/20 n NaOH = 6,1 mg Benzoesäure.

/8

1.2. Schwefelsäure 1/20 n

Aus einer Mikrobürette läßt man 10 ml der 1/20 n Schwefelsäure in einen 100 ml Erlenmeyerkolben einlaufen, gibt 5 Tropfen Tashiro-Indikator hinzu und bringt die Vorlage am Destillierapparat an. In den Destillierkolben gibt man 20 ml 25 %ige Natronlauge, verdünnt mit destilliertem Wasser bis zur Hälfte des Volumens und destilliert 7 bis 10 Minuten. Die Vorlage wird nun abgenommen, und, nachdem mit etwas destilliertem Wasser das in Schwefelsäure eintauchende Glasrohr abgespült wurde, titriert man mit 1/20 n Natronlauge von violett auf blaugrün. Der erhaltene Wert ist der Blindwert, der auch gleichzeitig eine Kontrolle des Destillier-Apparates ist und regelmäßig bestimmt wird. Diese Zahl wird der Berechnung des Stickstoffgehaltes zugrunde gelegt.

1.3. Tashiro-Indikator

0,24 g Methylrot in 300 ml Ethanol und 0,40 g Methylenblau in 400 ml 50 %igem Ethanol.

1.4. 25 %ige Natronlauge aus NaOH-Plätzchen.

1.5. Das zu prüfende Tensid wird als x-prozentige Lösung mit destilliertem Wasser angesetzt und auf pH 7 eingestellt.

2. Aufbereitung des Zein:

Das verwendete Zein aus Mais (MG 20.000 - 30.000; ~98,5 % Protein) wird im Vakuumtrockenschrank bei maximal 40° C bis zur Gewichtskonstanz getrocknet.

3. Durchführung des Tests:

In eine 50 ml-Enghalsflasche mit Schraubverschluß werden 40 ml Tensidlösung pipettiert und in einem Wasserbad auf 35° C temperiert. 2 g Zein werden auf einer Spielkarte genau eingewogen und durch einen Trichter aus Fotopapier schnell in die Flasche geschüttet und sofort 25 mal per Hand kräftig hin- und hergeschüttelt, damit sich das Zein gut verteilt und nicht zusammenballt.

In einem Wackerapparat, dessen Lufttemperatur auf 35° C eingestellt ist und der für diesen Zweck geeignete Stahleinsäzte mit Federn enthält, können mehrere Flaschen eingespannt werden, die nun 1 Stunde bei 5 U/min. rotieren. Anschließend wird ein aliquoter Teil der Lösung (ungefähr 25 ml) in einem Zentrifugenbecher aus V2A-Stahl eingewogen und 30 Minuten bei 2600 U/min. zentrifugiert. Nun werden ungefähr 10 ml durch einen Faltenfilter (Schleicher & Schüll, Nr. 597 1/2 Schwarzband Ø 5,5 cm) in einen 50 ml-Erlenmeyerkolben filtriert. Der Stickstoffgehalt dieser Lösung wird durch eine Doppelbestimmung nach der Mikro-Kjeldahl-Methode ermittelt.

1 ml der gelben Lösung (bei sehr schwach gefärbten Lösungen werden 2 bis 5 ml verwendet) wird in einem 100 ml Kjeldahl-Kolben mit einer kleinen Menge Selen-Reaktionsgemisch und ungefähr 3 ml konzentrierter Schwefelsäure versetzt. Der Kolben wird in das Elektro-Thermal-Gerät gebracht (die Kolben müssen außen sauber und trocken sein, ebenso darf keine Lösung in die Heizaggregate tropfen) und in der Hitze ungefähr 2 1/2 - 3 Stunden aufgeschlossen, bis die Flüssigkeit wäßrig klar ist.

Die abgekühlte Lösung wird nun in einen Destillierapparat überführt, mit 20 ml 25 %iger Natronlauge und einigen Tropfen Phenolphthalein versetzt, wobei darauf geachtet werden muß, daß die Probelösung auch wirklich alkalisch ist. Der Kolben darf nur ungefähr bis zur Hälfte gefüllt sein. Nun wird mit Wasserdampf in 7 - 10 Minuten (eventuell länger, mit pH-Papier prüfen) das Ammoniak in eine Vorlage mit 10 ml 1/20 n Schwefelsäure und circa 10 ml destilliertem Wasser überdestilliert. Durch Rücktitration der unverbrauchten Schwefelsäure in der Vorlage mit 1/20 n Natronlauge gegen Tashiro-Indikator wird der Säureverbrauch durch das überdestillierte Ammoniak und daraus die Stickstoffaufnahme berechnet:

$$1 \text{ ml } 1/20 \text{ n } H_2SO_4 \mathrel{\hat{=}} 0{,}7 \text{ mg N.}$$

Bei stickstoffhaltigen Tensiden wird der Gehalt an Stickstoff ebenfalls nach der Mikro-Kjeldahl-Methode bestimmt und bei der Ausrechnung vom Endergebnis abgezogen.

Bei dem Zein-Test wurden die in nachstehender Tabelle 1 aufgeführten Zein-Zahlen ermittelt, denen die sehr gute Hautfreundlichkeit der erfindungsgemäßen Tenside zu entnehmen ist.

Tabelle 1

| Substanz | Zein-Zahl |
|---|---|
| Nonylbenzolsulfonat 20 %ig | 620 |
| Natriumlaurylsulfat 20 %ig | 535 |
| Fettalkohol-$C_{12}$-$C_{14}$-ethersulfat mit 2 EO,20 %ig | 329 |
| Produkt A, 20 %ig | 223 |

Nachfolgend werden noch einige Beispiel für kosmetische Reinigungsmittel mit einem Gehalt an den erfindungsgemäßen Tensiden aufgeführt:

Schaumbad, klar

| | | |
|---|---|---|
| Produkt A | 30,0 | Gewichtsteile |
| Polyol-Fettsäureester, Cetiol HE(R) Rückfettungsmittel | 2,0 | " |
| Kokosfettsäurediethanolamid | 2,0 | " |
| Natriumchlorid | 3,0 | " |
| Parfümöl | 1,0 | " |
| Konservierungsmittel | 0,2 | " |
| Wasser | 61,8 | " |

Schaumbad, klar

| | | |
|---|---|---|
| Produkt B | 60,0 | Gewichtsteile |
| Polyol-Fettsäureester, Cetiol HE(R) Rückfettungsmittel | 4,0 | " |
| Kokosfettsäurediethanolamid | 1,0 | " |
| Natriumchlorid | 2,0 | " |
| Parfümöl | 2,0 | " |
| Konservierungsmittel | 0,2 | " |
| Wasser | 30,8 | " |

Duschbad, klar

| | | |
|---|---|---|
| Produkt A | 50,0 | Gewichtsteile |
| Polyol-Fettsäureester, Cetiol HE(R) Rückfettungsmittel | 3,0 | " |
| Kokosfettsäurediethanolamid | 2,0 | " |
| Natriumchlorid | 3,0 | " |
| Parfümöl | 1,0 | " |
| Konservierungsmittel | 0,2 | " |
| Wasser | 40,8 | " |

Duschbad, klar

| | | |
|---|---|---|
| Produkt B | 40,0 | Gewichtsteile |
| Ethoxylierter und propoxylierter gesättigter Fettalkohol, Aethoxal(R) | 10,0 | " |
| Natriumchlorid | 3,0 | " |
| Parfümöl | 3,0 | " |
| Konservierungsmittel | 0,2 | " |
| Wasser | 43,8 | " |

Körperwaschcreme für Tubenabfüllung

| | | |
|---|---|---|
| Produkt A | 52,0 | Gewichtsteile |
| Kokosfettsäuremonoethanolamid | 3,0 | " |
| Ethylenglykolstearat | 5,0 | " |
| Citronensäure | 0,05 | " |
| Parfümöl | 1,0 | " |
| Konservierungsmittel | 0,2 | " |
| Wasser | 38,75 | " |

Shampoo, klar

| | | |
|---|---|---|
| Produkt A | 40,0 | Gewichtsteile |
| Natriumchlorid | 4,0 | " |
| Parfümöl | 1,0 | " |
| Konservierungsmittel | 0,2 | " |
| Wasser | 54,8 | " |

Shampoo, klar

| | | |
|---|---|---|
| Produkt B | 50,0 | Gewichtsteile |
| Kokosfettsäurediethanolamid | 3,0 | " |
| Natriumchlorid | 0,5 | " |
| Parfümöl | 1,0 | " |
| Konservierungsmittel | 0,2 | " |
| Wasser | 45,3 | " |

Baby-Schaumbad

| | | |
|---|---|---|
| Produkt A | 50,0 | Gewichtsteile |
| Capryl-Caprinsäuretriglycerid | 15,0 | " |
| Kamillenextrakt | 3,0 | " |
| Parfümöl | 1,0 | " |
| Wasser | 31,0 | " |

/14

Patentansprüche:

1. Neuartige Tenside auf Basis von Salzen anionischer Tenside, dadurch gekennzeichnet, daß sie als Gegen-ion ein durch reduktive Aminierung reduzierender Mono-, Di- oder Oligosaccharide gewonnenes Zuckerderivat der allgemeinen Formel I enthalten,

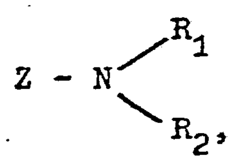

I

in der $R_1$ und $R_2$, die gleich oder verschieden sein können und Wasserstoff, einen Alkylrest oder Hydroxyalkylrest mit 1 - 4 Konlenstoffatomen darstellen, während Z einen Polyhydroxyalkylrest mit bis zu 7 Kohlenstoffatomen bedeutet, der gegebenenfalls mit einem Mono-, Di- oder Oligosaccharid glukosidisch verknüpft ist.

2. Neuartige Tenside nach Anspruch 1, dadurch gekennzeichnet, daß als anionische Tenside Alkylschwefelsäuremonoester, beziehungsweise Alkyletherschwefelsäuremonoester mit einer Alkylkette von 8 bis 18 Kohlenstoffatomen dienen.

3. Neuartige Tenside nach Anspruch 1, dadurch gekennzeichnet, daß als anionisches Tensid Fettalkohol-$C_{12}$-$C_{14}$-etherschwefelsäuremonoester mit ca. 2 Ethylenoxidgruppen im Molekül dient.

/15

4. Neuartige Tenside nach Anspruch 1 - 3, dadurch gekennzeichnet, daß das als Gegenion dienende Zuckerderivat von der Glucose abgeleitet ist und insbesondere N-Methylglucamin, N-β-Hydroxyethylglucamin beziehungsweise N-β-Hydroxyethyl-N-methylglucamin ist.

5. Herstellung der neuartigen Tenside nach Anspruch 1 - 4, dadurch gekennzeichnet, daß man die nach allgemein bekannten Verfahren durch reduktive Aminierung reduzierender Mono-, Di- oder Oligosaccharide gewonnenen Zuckerderivate mit der äquivalenten Menge des sauren anionischen Tensids neutralisiert.

6. Verwendung der neuartigen Tenside gemäß Anspruch 1 - 5 als alleinigen tensidischen Anteil oder im Gemisch mit anderen Tensiden in hautfreundlichen kosmetischen Reinigungsmitteln.

7. Hautfreundliche kosmetische Reinigungsmittel mit einem Gehalt an den neuartigen Tensiden gemäß Anspruch 1 - 5 als alleinigen tensidischen Anteil, beziehungsweise im Gemisch mit anderen Tensiden.

8. Hautfreundliche kosmetische Reinigungsmittel nach Anspruch 7, dadurch gekennzeichnet, daß sie die neuartigen Tenside in einer Menge von 10 - 70 Gewichtsprozent, bezogen auf das gesamte Mittel, enthalten.

Patentansprüche für Österreich

1. Verfahren zur Herstellung neuartiger Tenside auf Basis von Salzen anionischer Tenside, dadurch gekennzeichnet, daß man durch reduktive Aminierung reduzierender Mono-, Di- oder Oligosaccharide gewonnene Zuckerderivate der allgemeinen Formel I

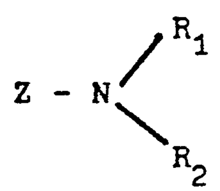

I

in der $R_1$ und $R_2$, die gleich oder verschieden sein können und Wasserstoff, einen Alkylrest oder Hydroxyalkylrest mit 1-4 Kohlenstoffatomen darstellen, während Z einen Polyhydroxyalkylrest mit bis zu 7 Kohlenstoffatomen bedeutet, der gegebenenfalls mit einem Mono-, Di- oder Oligosaccharid glucosidisch verknüpft ist, mit einer äquivalenten Menge eines sauren anionischen Tensids neutralisiert.

2. Verfahren nach Anspruch 1, dadurch gekennzeichnet, daß als anionische Tenside Alkylschwefelsäuremonoester beziehungsweise Alkyletherschwefelsäuremonoester mit einer Alkylkette von 8 bis 18 Kohlenstoffatomen verwendet werden.

3. Verfahren nach Anspruch 1 und 2 dadurch gekennzeichnet, daß als anionisches Tensid Fettalkohol-$C_{12-14}$-etherschwefelsäuremonoester mit ca. 2 Ethylenoxidgruppen im Molekül verwendet wird.

...

4. Verfahren nach Anspruch 1 bis 3, dadurch gekennzeichnet, daß man ein Glucosederivat und insbesondere N-Methylglucamin, N-ß-Hydroxyethylglucamin, beziehungweise N-ß-Hydroxyethyl-N-methylglucamin mit einer äquivalenten Menge eines sauren, anionischen Tensids neutralisiert.

5. Verwendung von nach Anspruch 1 bis 4 hergestellten neuartigen Tensiden als alleinigem tensidischen Anteil oder im Gemisch mit anderen Tensiden in hautfreundlichen kosmetischen Reinigungsmitteln.

6. Hautfreundliche kosmetische Reinigungsmittel mit einem Gehalt an neuartigen, nach Anspruch 1 bis 4 hergestellten Tensiden als alleinigem tensidischen Anteil, beziehungsweise im Gemisch mit anderen Tensiden.

7. Hautfreundliche kosmetische Reinigungsmittel nach Anspruch 6, dadurch gekennzeichnet, daß sie neuartige Tenside, hergestellt nach Anspruch 1 bis 4, in einer Menge von 10 - 70 Gewichtsprozent bezogen auf das gesamte Mittel, enthalten.

Europäisches Patentamt

# EUROPÄISCHER RECHERCHENBERICHT

0039860

Nummer der Anmeldung

EP 81 10 3330

| Kategorie | EINSCHLÄGIGE DOKUMENTE — Kennzeichnung des Dokuments mit Angabe, soweit erforderlich, der maßgeblichen Teile | betrifft Anspruch |
| --- | --- | --- |
| X | H. JANISTYN: "Handbuch der Kosmetika und Riechstoffe", 3. Auflage, Band 1, 1978, Seite 423 Dr. Alfred Hüthig Verlag Heidelberg, DE. "Die kosmetischen Grundstoffe" * Seite 423, "Glucamin" * | 1,5,6 |
| | -- | |
| | FR - A - 2 151 350 (UNILVER-EMERY) * Seite 11, Beispiel 8, "Crème de nettoyage" * & DE - A - 2 241 261 | 1,5-7 |
| | -- | |
| | US - A - 2 091 704 (DUNCAN et al.) * Insgesamt * | 1,2,5-7 |
| | -- | |
| | PATENT ABSTRACTS OF JAPAN, Band 4, Nr. 12, (C-71), 29. Januar 1980, Seite 106C71 Tokyo, JP. & JP - A - 54 147 937 (KANEBO) (19-11-1979) * Zusammenfassung * | 1,6-8 |
| | -- | |
| | GB - A - 428 142 (DU PONT DE NEMOURS) * Seite 1, Zeilen 49-88; Seite 3, Zeile 128 - Seite 4, Zeile 15 * | 1,2 |
| | -- | |
| A | AU - B - 476 632 (VAN DIJK) | |
| | ---- | |

KLASSIFIKATION DER ANMELDUNG (Int. Cl.³)

A 61 K 7/08
7/50
C 11 D 1/42

RECHERCHIERTE SACHGEBIETE (Int Cl.³)

A 61 K 7/08
7/50
C 11 D 1/14
1/38
1/40
1/42
1/65

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
| --- | --- | --- |
| Den Haag | 17-08-1981 | BENZ |